# EUROPEAN PATENT APPLICATION

(11) **EP 4 693 318 A1**
(43) Date of publication of application: **11.02.2026**
(21) Application number: 25190722.6
(22) Date of filing: 21.07.2025
(51) Int. Cl.: G16H 10/40, G06N 20/00, G06Q 10/0639, G16H 40/40

(54) **METHOD, DEVICE FOR RECOMMENDING QUALITY CONTROL RULES AND COMPUTER EQUIPMENT**

(30) Priority: 29.07.2024 CN 202411026805
(71) Applicant: Shenzhen New Industries Biomedical Engineering Co., Ltd., Shenzhen, Guangdong (CN)
(72) Inventor: TANG, Junhui, Shenzhen (CN); CHEN, Jiale, Shenzhen (CN); LIANG, Chuanlin, Shenzhen (CN); LI, Peipei, Shenzhen (CN); PAN, Xiong, Shenzhen (CN)
(74) Representative: Metida

(57) **Abstract**

The present application relates to a method, a device for recommending quality control rules and a computer equipment. The method includes: displaying a recommendation interface, and the recommendation interface comprising an information display area and a rule recommendation area; displaying a target quality control material in the information display area; obtaining at least one quality control recommendation rule recommended for the target quality control material; displaying the at least one quality control recommendation rule for the target quality control material in the rule recommendation area of the recommendation interface. By providing a recommendation interface and displaying at least one quality control recommendation rule recommended for the target quality control material on the recommendation interface, the method eliminates manual calculation of internal quality control rules by users, significantly enhancing both the accuracy and efficiency of quality control rule configuration.

## Description

### CROSS-REFERENCE TO RELATED APPLICATIONS

This application claims the priority to Chinese Patent Application No. 202411026805.3 filed on July 29, 2024, entitled "Method, device for recommending quality control rules, and computer equipment", in China National Intellectual Property Administration, the contents of which are incorporated by reference herein.

### FIELD

The present application relates to a technical field of medical testing and internal quality control, and in particular to a method, a device for recommending quality control rules, and a computer equipment.

### BACKGROUND

With the advancement of medical technology, an increasing number of medical testing equipment and medical detection methods are being applied in laboratories. Meanwhile, there are higher demands for the accuracy and reliability of test results.

In order to ensure the accuracy and reliability of laboratory testing, a default method is internal quality control. Internal quality control is a quality control method designed to monitor and evaluate the performance of a laboratory, ensuring the accuracy of routine test reports. Wherein, quality control rules are the core component of internal quality control, serving as a set of criteria to determine whether test results are acceptable. Therefore, establishing and implementing appropriate quality control rules is essential for guaranteeing the reliability of test results.

Conventionally, internal quality control rules require manual calculations by users, a process that is not only cumbersome but also influenced by the user's experience. This may lead to mismatches between the internal quality control rules and the quality control materials used.

### SUMMARY

Based on the above problem, it is necessary to provide a method, a device for recommending quality control rule, a computer equipment, a computer readable storage medium and computer program product to address the above technical problems, which can improve the accuracy and efficiency of internal quality control rule configuration.

In a first aspect, the present application provides a method for recommending quality control rules, the method includes:
displaying a recommendation interface, and the recommendation interface comprising an information display area and a rule recommendation area;
displaying a target quality control material in the information display area;
obtaining at least one quality control recommendation rule recommended for the target quality control material;
displaying the at least one quality control recommendation rule for the target quality control material in the rule recommendation area of the recommendation interface.

In one of the embodiments, the method further includes: obtaining a recommendation rationale for each quality control recommendation rule;
displaying the at least one quality control recommendation rule for the target quality control material in the rule recommendation area of the recommendation interface, comprises:
displaying the at least one quality control recommendation rule for the target quality control material and the recommendation rationale corresponding to each quality control recommendation rule in the rule recommendation area.

In one embodiment, the information display area includes a plurality of levels of attribute selection bars of a quality control material;
displaying the target quality control material in the information display area, includes:
in response to a selection operation on the attribute selection bar of each level, displaying selected attributes of each level in the attribute selection bar of each level;
determine the target quality control material according to the selected attributes of each level.

In one embodiment, the target quality control material includes at least one of the following types:
a first type, indicating that the target quality control material is a quality control material with abnormal internal quality control rules;
a second type, indicating that the target quality control material is the quality control material whose sigma value of internal quality control is lower than a first threshold;
a third type, indicates that the target quality control material is the quality control material whose sigma value of internal quality control is lower than a second threshold and has not increased within a preset time period.

In one of the embodiments, the recommendation interface also includes a recommendation control, obtaining at least one quality control recommendation rule recommended for the target quality control material, includes:
in response to a trigger operation on the recommendation control in the recommendation interface, obtaining the at least one quality control recommendation rule for the target quality control material.

In one of the embodiments, the recommendation interface further includes a parameter configuration area, and the parameter configuration area comprises a plurality of quality control parameters to be set;
the method further includes:
obtaining a configuration operation in the parameter configuration area of the recommendation interface, and obtaining a plurality of the quality control parameters;
in response to the trigger operation on the recommendation control in the recommendation interface, obtaining the at least one quality control recommendation rule for the target quality control material, comprises:
in response to a triggering operation on the recommendation control in the recommendation interface, obtaining the at least one quality control recommendation rule for the target quality control material based on the plurality of quality control parameters.

In one of the embodiments, the recommendation interface further includes an analysis result area;
the method further includes:
obtaining a quality control analysis result analyzed by quality control recommendation; and the quality control analysis result comprising a function graph of a plurality of quality control rules and an operation process specification graph; and the quality control analysis result being used to determine the at least one quality control recommendation rule;
displaying the quality control analysis result in the analysis result area.

In one of the embodiments, the recommendation interface further includes a rule configuration control;
the method further comprises:
obtaining a first target quality control rule selected from the at least one quality control recommendation rule;
in response to a triggering operation on the rule configuration control in the recommendation interface, configuring the first target quality control rule as an internal quality control rule for the target quality control material.

In one of the embodiments, the recommendation interface further includes a plurality of rule configuration controls corresponding to each of quality control recommendation rules;
the method further includes:
in response to a triggering operation on a rule configuration control of the recommendation interface, obtaining a first target quality control rule corresponding to the rule configuration control;
configuring the first target quality control rule as an internal quality control rule of the target quality control material.

In one embodiment, configuring the first target quality control rule as the internal quality control rule of the target quality control material includes:
displaying a rule configuration interface;
configuring the first target quality control rule as the internal quality control rule of the target quality control material in the rule configuration interface.

In one embodiment, the internal quality control rule includes an out-of-control rule, and the rule configuration interface comprises a default configuration area, and the default configuration area comprises a first quality control rule list, and the first quality control rule list comprises all quality control rules;
configuring the first target quality control rule as the internal quality control rule of the target quality control material in the rule configuration interface, comprises:
configuring the first target quality control rule in the first quality control rule list as the out-of-control rule for the target quality control material in the default configuration area.

In one embodiment, the rule configuration interface further includes a quick configuration area, and the quick configuration area comprises a second quality control rule list, and the second quality control rule list comprises at least one quality control recommendation rule, and at least one operation control corresponding to the quick configuration method for each of the quality control recommendation rules, the method further includes:
in response to an operation on the operation control of any of the quality control recommendation rules in the quick configuration area, obtaining a second target quality control rule and a quick configuration method, and the quick configuration method comprising at least one of an overwriting method and an appending method;
in response that the quick configuration method is the overwriting method, then in the conventional configuration area, cancelling current out-of-control rule in the first quality control rule list, and updating the second target quality control rule to the out-of-control rule of the target quality control material;
in response that the quick configuration method is the appending method, then in the conventional configuration area, appending the second target quality control rule in the second quality control rule list as the out-of-control rule for the target quality control material.

In a second aspect, the present application also provides a device for recommending quality control rule recommendation, the device includes:
a display module, used to display a recommendation interface, and the recommendation interface comprising an information display area and a rule recommendation area;
a quality control material processing module, used to display a target quality control material in the information display area;
a recommendation module, used to obtain at least one quality control recommendation rule recommended for the target quality control material;
a rule display module, used to display at least one quality control recommendation rule of the target quality control material in the rule recommendation area of the recommendation interface.

In a third aspect, the present application further provides a computer equipment, comprising a memory and a processor, wherein the memory stores computer programs, wherein the processor implements steps of the method for recommending quality control rule according to each of the above embodiments when executing the computer programs.

In a fourth aspect, the present application further provides a computer-readable storage medium having computer programs stored thereon, which, when executed by a processor, implements steps of the method for recommending quality control rule according to each of the above embodiments.

In a fifth aspect, the present application further provides a computer program product, which includes computer programs, and when the computer programs are executed by a processor, the steps of the method for recommending quality control rule according to each of the above embodiments are implemented.

The above-mentioned method, device for recommending quality control rule, computer equipment, storage medium and computer program product provide a recommendation interface, which displays at least one quality control recommendation rule recommended for the target quality control material, thereby eliminating the need for users to manually calculate internal quality control rules, thereby improving the accuracy and efficiency of internal quality control rule configuration.

### BRIEF DESCRIPTION OF THE DRAWINGS

FIG. 1 is an application environment diagram of a method for recommending quality control rules in one embodiment;
FIG. 2 is a schematic flow chart of the method for recommending quality control rules in one embodiment;
FIG. 3 is a schematic diagram of a recommendation interface in one embodiment;
FIG. 4 is a schematic diagram of a rule configuration interface in one embodiment;
FIG. 5 is a schematic diagram of an application object configuration area in one embodiment;
FIG. 6 is a schematic diagram of a rule configuration interface in another embodiment;
FIG. 7 is a structural block diagram of a device for recommending quality control rules in one embodiment;
FIG. 8 is a diagram showing the internal structure of a computer device in one embodiment.

### DETAILED DESCRIPTION

In order to make the purpose, technical solution and advantages of the present application more clearly understood, the present application is further described in detail below in conjunction with the accompanying drawings and embodiments. It should be understood that the specific embodiments described herein are only used to explain the present application and are not used to limit the present application.

Internal quality control is crucial for clinical testing. Internal quality control is an essential function to be implemented in Laboratory Information Systems. It refers to a series of inspection and control measures adopted by laboratories to monitor and evaluate their operational quality, determining whether routine test reports can be issued reliably.

Common methods of internal quality control include a standard material method, where in a laboratory, measurements are performed by using quality control materials to evaluate the sensitivity, precision, and accuracy of the testing system. Internal quality control uses quality control rules to determine whether the instrument status is stable and whether there is a "out of control" phenomenon. Quality control rules include Westgard rules, among which Westgard rules include 1_2S, 1_3S, 2_2S and other rules.

Typically, in each laboratory, the internal quality control rules for the quality control materials of each experimental project are configured. In order to improve the accuracy and efficiency of internal quality control rule configuration, the present application provides a method for recommending quality control rule, and the method includes: displaying a recommendation interface, and the recommendation interface including an information display area and a rule recommendation area; displaying a target quality control material in the information display area; obtaining at least one quality control recommendation rule recommended for the target quality control material; and displaying at least one quality control recommendation rule for the target quality control material in the rule recommendation area of the recommendation interface. By adopting this method, a recommendation interface is provided to display at least one recommended quality control rule for the target control material, thereby eliminating the need for users to manually calculate the internal quality control rules, thereby improving the accuracy and efficiency of internal quality control rule configuration.

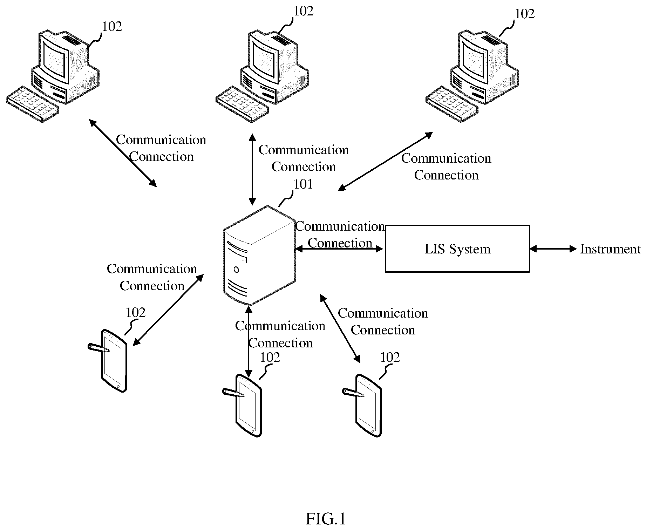

Specifically, the present application provides a cloud-based quality control system, whose foundational architecture is shown in FIG. 1. The system includes an application client 102, and a backend service 101 that provides data processing for the application client 102.

In one embodiment, the backend service 101 is connected to a LIS system. The LIS system is a hospital laboratory information management system, which mainly manages various services of the hospital laboratories, including sample management, test process management, test result management, report management, etc.

In one embodiment, the application client 102 obtains the quality control results of the instrument and the sample number used to identify the quality control from the LIS system through the backend service 101, and uploads the quality control results to the backend service 101. The backend service 101 can perform internal quality control (IQC), internal quality control inter-laboratory and external quality assessment (EQA)based on the quality control results. The cloud-based quality control system is based on the provision of laboratory quality control data analysis services, and more comprehensively reflects the accuracy and precision of the laboratory through three key dimensions: IQC, internal quality control inter-laboratory, and EQA. The quality control data is intelligently analyzed with paperless multi-charts, allowing users to locate systematic errors or random errors more quickly.

In one embodiment, the application client 102 can be a personal computer, a laptop computer, and a mobile terminal, etc. The mobile terminal can communicate with the backend service 101, and laboratory staff or staff of the cloud-based quality control system can log in to the cloud-based quality control system through the mobile terminal to perform corresponding management and settings.

In one embodiment, the backend service 101 of the cloud-based quality control system may be implemented by an independent server or a server cluster consisting of multiple servers.

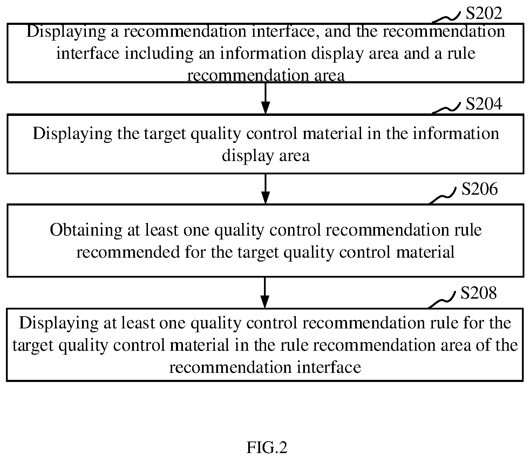

In one embodiment, as shown in FIG. 2, a method for recommending quality control rule is provided, which is described by taking the method applied to the application client in FIG. 1 as an example, and includes the following steps.

Step 202, displaying a recommendation interface, and the recommendation interface including an information display area and a rule recommendation area.

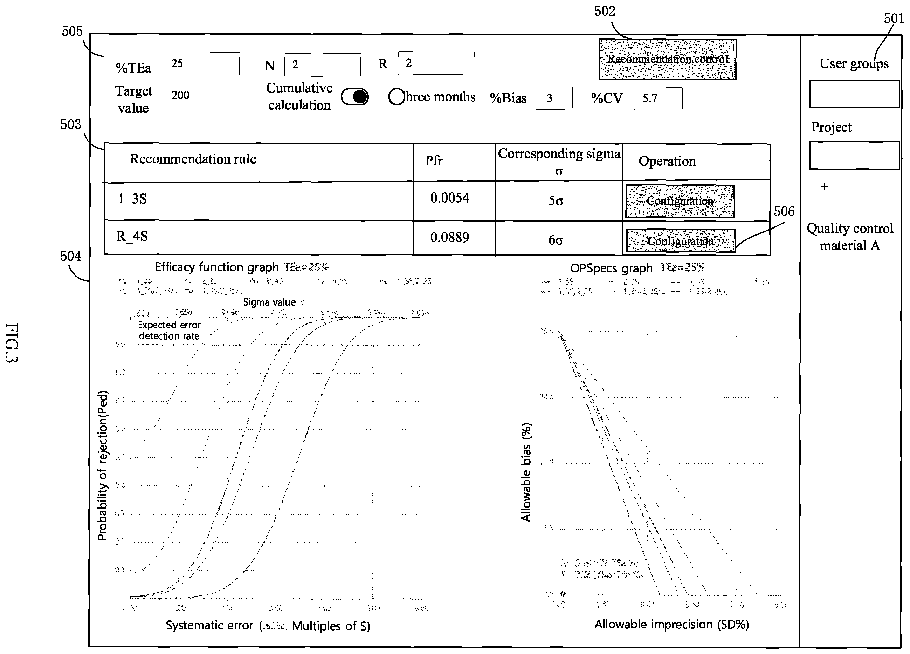

The recommendation interface is an application interface of the internal quality control application, through which the user can use the recommendation function of the quality control rules. The recommendation interface of an embodiment is shown in FIG. 3, and the recommendation interface may include the information display area 501 and the rule recommendation area 503. In one embodiment, the information display area 501 is used to display the quality control material information, and the rule recommendation area 503 is used to display the recommended quality control rules.

Step 204, displaying the target quality control material in the information display area.

The quality control materials are specimens or solutions used for quality control purposes. They usually have known properties or concentrations and are used to evaluate the performance of laboratory test methods or instruments. By comparing the quality control results of quality control materials with known values, the accuracy and reliability of their test methods and instruments can be determined in the laboratories. Quality control materials can be of various types, such as assayed controls, unassayed controls, etc.

In one embodiment, the information display area 501 provides an interactive control, and the user inputs the information of the target quality control material by operating the interactive control, and the target quality control material is displayed in the information display area. In one embodiment, the target quality control material can be located according to a predefined hierarchical relationship. In one embodiment, the target quality control material can be located according to user group-project-quality control material. The user group corresponds to the experimental group, the project corresponds to the detection project under a certain experimental group, and the target quality control material is the quality control material of a certain detection project under a certain experimental group.

In one embodiment, the application client monitors the quality control results of each quality control material, enabling timely detection of target quality control materials with abnormal internal quality control rule violations. In one embodiment, the backend service can also be used to monitor the quality control results of multiple quality control materials in the experimental group, so as to timely detect target quality control material with abnormal internal quality control rule violations, and send the target quality control material with abnormal internal quality control rules to the application client to serve as a prompt.

Step 206, obtaining at least one quality control recommendation rule recommended for the target quality control material.

Specifically, the application client may determine at least one quality control recommendation rule for the target quality control material through a preset quality control rule recommendation algorithm. Alternatively, the backend service may determine at least one quality control recommendation rule for the target quality control material through a preset quality control rule recommendation algorithm, and send the at least one quality control recommendation rule recommended for the target quality control material to the application client, so that the application client may obtain the at least one quality control recommendation rule recommended for the target quality control material.

In one embodiment, in order to improve the accuracy of the recommendation, the preset quality control rule recommendation algorithm can use a plurality of quality control rules to traverse, and obtain the quality control analysis results of each quality control rule, and determine the top N quality control rules with the best quality control effects in the plurality of quality control analysis results as the quality control recommendation rules.

The quality control rule recommendation algorithm can determine the quality control parameters through historical data of the target quality control material or the screening similar quality control materials. The system analyzes according to the quality control parameters and fits the analysis results of the historical data using various rules. The analysis results include the quality control of the historical data using various rules and the quantitative value of the quality control effect corresponding to each rule. In other words, this method uses a traversal method to traverse various rules to perform quality control analysis on historical data and obtain the quality control analysis results of each quality control rule. The quality control effects of each quality control rule can be compared through the quantitative value of the quality control effect, and at least one quality control recommendation rule can be determined with reference to the comparison results of the quantitative value of the quality control effect.

Step 208: displaying at least one quality control recommendation rule for the target quality control material in the rule recommendation area of the recommendation interface.

In one embodiment, the rule recommendation area of the recommendation interface is used to display the quality control rules recommended for the target quality control material, that is, to display the quality control recommendation rules for the target quality control material.

The above method for recommending quality control rule provides a recommendation interface, which displays at least one quality control recommendation rule recommended for the target quality control material, thereby eliminating the need for users to manually calculate the internal quality control rules, thereby improving the accuracy and efficiency of internal quality control rule configuration.

In another embodiment, the method for recommending quality control rule further includes: obtaining a recommendation rationale for each quality control recommendation rule. In the rule recommendation area, at least one quality control recommendation rule for the target quality control material and the recommendation rationale corresponding to each quality control recommendation rule are displayed.

Namely, while obtaining at least one quality control recommendation rule recommended for the target quality control material, the recommendation rationale of each quality control recommendation rule is also obtained.

The recommendation rationale of the quality control recommendation rule refers to the reason for recommending the quality control rule as the quality control rule of the quality control material. For example, the recommendation rationale may include the reason for recommendation. By obtaining and displaying the recommendation rationale of the quality control recommendation rule, the user can know why the quality control recommendation rule is recommended, thereby increasing the credibility of the quality control recommendation rule. In addition, according to the recommendation rationale of each quality control recommendation rule, the user can select the most suitable quality control recommendation rule according to the recommendation rationale.

In one embodiment, the recommendation rationale may be the quantitative value of the quality control effect of the quality control rule. Based on the quantitative value of the quality control effect, the user can know the quality control effect of each quality control recommendation rule and the difference in quality control effects between different quality control recommendation rules. Thus, the user can select the most suitable quality control recommendation rule based on the quantitative value of the quality control effect of the quality control recommendation rule.

In one embodiment, as shown in FIG. 3, the quantitative value of the quality control effect can be at least one of a sigma value and a probability for false rejection (Pfr). Taking the sigma value as an example, a value greater than or equal to 6 σ is world-class, and a value greater than or equal to 5 σ is excellent. Taking the probability for false rejection as an example, in clinical testing, the probability for false rejection should not exceed 5%. At least one of the sigma value and the probability for false rejection is used to characterize the quantitative value of the quality control effect to ensure the reliability of the quality control system and the credibility of the experimental data.

After obtaining the quality control recommendation rules and the recommendation rationale of the quality control recommendation rules, the application client also displays at least one quality control recommendation rule for the target quality control material and the recommendation rationale corresponding to each quality control recommendation rule in the rule recommendation area.

In one embodiment, for the convenience of user viewing, at least one quality control recommendation rule for the target quality control material and the recommendation rationale corresponding to each quality control recommendation rule can be displayed in the form of a list. The list can include a plurality of rows, each row is used to record the information of a quality control recommendation rule, including the quality control recommendation rule and the recommendation rationale.

In the embodiment, by obtaining the recommendation rationale of the quality control recommendation rule, the credibility of the quality control recommendation rule can be improved, and a rationale can be provided for the user to select the quality control rule.

In another embodiment, the information display area includes multiple levels of attribute selection bars for the quality control material. Displaying the target quality control material in the information display area includes: in response to a selection operation on the attribute selection bar of each level, displaying the selected attributes of each level in the attribute selection bar of each level; and determining the target quality control material according to the set attributes of each level.

The recommendation interface of an embodiment is shown in FIG. 3. The recommendation interface includes an information display area 501, in which a plurality of levels of attribute selection bars can be displayed. As shown in FIG. 3, according to the relationship between the upper and lower levels of the attributes, the attribute selection bar can include a user group selection bar for selecting user group attributes, a project selection bar for selecting project attributes, and a quality control material name selection bar for selecting quality control material name attributes.

After entering the recommendation interface, the user can manually set in the information display area 501 to determine the target quality control material for quality control rule recommendation. For example, according to the user setting "A user group - B project - C quality control material", the target quality control material is determined.

In the embodiment, a method is provided for a user to manually select a target quality control material for which quality control rule recommendation is to be performed.

In other embodiments, to facilitate user operation, the system may also monitor target quality control material that meet the requirements in the background, and display the target quality control material information in the information display area 501 after entering the recommendation interface.

In one embodiment, the target quality control material is a quality control material with abnormal internal quality control rules.

Specifically, the internal quality control rule abnormality refers to the situation where the quality control result of the quality control material is inaccurate when the internal quality control rule is used to control the quality control material. It can be understood that whether it is the application client or the internal quality control rule adopted by the backend service, it is configured in advance by the staff through the rule configuration interface. The application client or the backend service performs quality control on the quality control material according to the configured internal quality control rule to determine whether there is an "out of control" phenomenon. Concurrently, the application client or the backend service can also automatically analyze and verify the internal quality control rules used by the quality control material to verify whether the current internal quality control rules are used appropriately. Alternatively, the internal quality control rules used by the quality control material can also be manually analyzed and verified to verify whether the current internal quality control rules are used appropriately. When it is verified that the internal quality control rules used by the quality control material are inappropriate, resulting in the judgment that the quality control result is out of control or inaccurate, it is determined that the internal quality control rules of the quality control material are abnormal, and the quality control material is determined as the target quality control material.

Therefore, when a quality control material with abnormal internal quality control rules is detected, it is determined as a target quality control material. When the user opens the recommendation interface, the target quality control material is displayed in the information display area of the recommendation interface, and quality control rules are recommended for the target quality control material.

In one embodiment, the target quality control material is a quality control material whose sigma value of internal quality control is lower than a first threshold. If the sigma value of the laboratory's internal quality control is lower than the first threshold, an abnormal prompt may be given, and the quality control material may be determined as the target quality control material. In one embodiment, the first threshold may be an empirical value.

Furthermore, the target quality control material is a quality control material whose sigma value of internal quality control is lower than the second threshold and has not increased within a preset time period. Specifically, in the laboratory, the quality control situation is generally a constant focus, and the sigma value hopes to be continuously improved. If the sigma value of the laboratory has not increased for a period of time and has not reached the laboratory's required level (the second threshold), it can be determined as a target quality control material, and an abnormal prompt will be issued to recommend quality control rules for it.

In one embodiment, when the information display area 501 displays a plurality of target quality control materials, the user selects the target quality control material for which quality control rule recommendation is to be performed.

The system monitors target quality control materials that need quality control rule recommendations in the background, and obtains at least one quality control recommendation rule recommended for the target quality control material. By displaying at least one quality control recommendation rule for the target quality control material in the rule recommendation area of the recommendation interface, the system can actively monitor the target quality control material whose quality control effect does not meet expectations, allowing the user to visually perceive the fact that the internal quality control rules of the target quality control material do not meet expectations. At the same time, the system automatically provides at least one quality control recommendation rule for the target quality control material without requiring the user to perform complex calculations, helping the user modify the internal quality control rules of the target quality control material in time, so that the internal quality control rules of the target quality control material can be corrected in time, thereby improving the accuracy of quality control.

In one embodiment, as shown in FIG. 3, a recommendation control 502 is also displayed on the recommendation interface to prompt the user that the recommendation function can be used to recommend quality control rules for the target quality control material.

The user determines the target quality control material through the information display area 501 of the recommendation interface, the user can click the recommendation control 502. The application client responds to a trigger operation of the recommendation control 502 on the recommendation interface and obtains at least one quality control recommendation rule for the target quality control material. In one embodiment, the application client uses a preset recommendation method to determine at least one quality control recommendation rule for the target quality control material. In another embodiment, the application client responds to the trigger operation of the recommendation control 502 on the recommendation interface and sends a recommendation request to the quality control cloud server. The quality control cloud server responds to the recommendation request and determines at least one quality control recommendation rule for the target quality control material according to the preset recommendation method. The quality control cloud server sends the determined at least one quality control recommendation rule to the application client, so that the application client obtains at least one quality control recommendation rule for the target quality control material.

As shown in FIG. 3, the recommendation interface also includes a rule recommendation area 503, and the rule recommendation area 503 displays at least one quality control recommendation rule for the target quality control material, so that the user can know the target quality control material with abnormal internal quality control rules in a timely manner, and the quality control recommendation rules can be pushed by the system without complicated calculations.

The method integrates a rule recommendation area, an information display area, and a recommendation control in a recommendation interface, so that the target quality control material can be determined in one interface, and after triggering a recommendation operation for the target quality control material, the quality control recommendation rule for the target quality control material can be obtained, which can reduce the cost and operation of user switching and improve convenience.

In another embodiment, in order to improve the accuracy of the recommendation, the preset quality control rule recommendation algorithm can use a plurality of quality control rules to traverse, obtain the quality control analysis results of each quality control rule, and determine the top N quality control rules with the best quality control effect and that meet the requirements among the plurality of quality control analysis results as the quality control recommendation rules.

In order to obtain the matching quality control recommendation rules, a recommendation interface of an embodiment is shown in FIG. 3, and may also include a configuration area 505, and the configuration area 505 is provided to the user to configure a plurality of quality control parameters.

In one embodiment, the quality control parameters may include TEa, N, and R, wherein N represents the number of analysis batches; TEa represents a total allowable error, that is, the quality specification; and R represents the number of quality control results.

In one embodiment, the quantitative value of the quality control effect can be a sigma value, which is a standard for measuring the experimental performance evaluation. The sigma value can be calculated by a formula, σ=(TEa-Bias)/CV, wherein TEa is the total allowable error (quality indicator), Bias is the bias (a type of systematic error), and CV is the coefficient of variation. After the user configures the quality control parameters, in response to the trigger operation for the recommended control in the recommendation interface, at least one quality control recommendation rule for the target quality control material is obtained based on the plurality of quality control parameters.

In one embodiment, Bias and CV can be obtained through two methods. The first method includes: after entering a target value, selecting a historical data period, and automatically calculating the Bias and the CV. The other method includes: manually entering the Bias and CV directly.

In another embodiment, in order to improve the convenience of setting the quality control parameters, the initial values of the quality control parameters of the configuration area can be first obtained through system recommendation. Based on the quality control parameters recommended by the system, the user can further modify the settings in combination with actual needs. In one embodiment, the quality control parameters can be learned and derived by the system from historical data.

Specifically, after obtaining relevant information about the target quality control material, instrument, and instrument location, the system matches similar quality control materials with optimal and stable quality control effects under the same conditions based on the above information, obtains the quality control parameters of similar quality control materials, and uses them as the quality control parameters of the target quality control material, thereby reducing the time users spend on setting quality control parameters and improving efficiency.

In the embodiment, by providing a configuration area on the recommendation interface, it is convenient for users to set quality control parameters, thereby improving the accuracy of quality control rule recommendations.

In another embodiment, the recommendation interface further includes an analysis result area 504. The method for recommending quality control rule further includes: obtaining quality control analysis results for quality control recommendation analysis; the quality control analysis results including a plurality of efficacy function graphs of quality control rule and an operation process specification graph; and displaying the quality control analysis results in the analysis result area.

Specifically, the quality control analysis results are used to represent the process and results of the quality control rule analysis.

Typically, obtaining quality control recommendation rules mainly includes three steps: 1.collecting quality control parameters; 2.calculating relevant data, and drawing the efficacy function graph and the operation process specification graph; 3.recommending quality control rules based on the relevant data, the efficacy function graph and the operation process specification graph. Specifically, after entering the TEa, N, R, Bias and CV of each project, relevant data such as sigma value σ, false rejection rate Pfr, probability of rehection Ped, etc., are calculated, and the operation process specification graph (OPSpecs graph) and efficacy function graph are drawn, and the sigma value is combined to select the quality control rules that meet the requirements as the quality control recommendation rules.

In one embodiment, in order to compare the quality control rules, different quality control rules in the figure (FIG. 3) can be represented by lines with different display parameters, and the legend of each quality control rule can be displayed in the figure. When clicking the corresponding legend or line in the figure, the line corresponding to the corresponding quality control rule can be hidden or displayed, which is convenient for users to flexibly choose which quality control rule lines to display on the figure, and provides convenience for users to compare the quality control effects of different quality control rules.

In order to improve the confidence of the quality control rule recommendation, the present embodiment provides the quality control analysis results, which include the efficacy function graphs of a plurality of quality control rules and the operation process specification graph, and displays the quality control analysis results in the analysis result area. Thus, the user can understand the process of how the quality control recommendation rules are determined through the quality control analysis results, thereby improving the confidence of the quality control analysis.

In another embodiment, the recommendation interface further includes a rule configuration control 506. The method for recommending quality control rule further includes: obtaining a first target quality control rule selected from at least one quality control recommendation rule; in response to a trigger operation on the rule configuration control in the recommendation interface, configuring the first target rule as the quality control rule of the target quality control material.

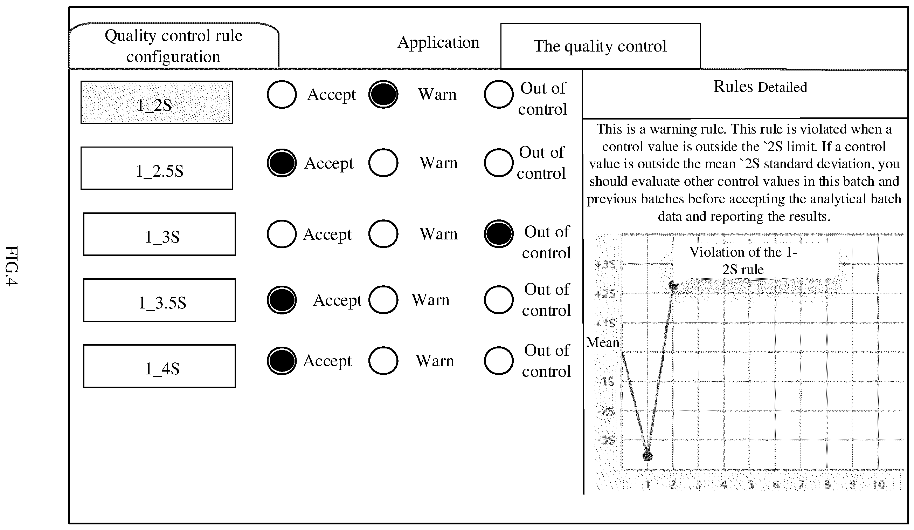

Typically, configuring quality control rules requires accessing the rule configuration interface shown in FIG. 4. Even after the user obtains the quality control recommendation rules recommended by the system, in order to use the quality control recommendation rules, the user needs to remember the quality control recommendation rules and the target quality control materials, and then switch from the recommendation interface to the rule configuration interface for configuration.

In the embodiment, a rule configuration control 506 is also provided in the recommendation interface to facilitate user operation. The rule configuration control is used to trigger configuration of the recommendation rule to the target quality control material, so that the quality control recommendation rule can be used to quickly configure the quality control rule in the recommendation interface, without the need for the user to remember the rule and then switch to the configuration interface for complex operations.

In one embodiment, the recommendation interface further includes a rule configuration control. A first target quality control rule selected from at least one quality control recommendation rule is obtained; in response to a trigger operation on the rule configuration control in the recommendation interface, the first target quality control rule is configured as an internal quality control rule for the target quality control material. In the embodiment, after selecting the first target quality control rule, the user clicks the rule configuration control to configure the first target quality control rule as the internal quality control rule for the target quality control material.

In one embodiment, as shown in FIG. 3, a rule configuration control 506 may be set, In one embodiment each quality control rule corresponds to a rule configuration control 506. In response to a trigger operation on the rule configuration control of the recommendation interface, a first target quality control rule corresponding to the rule configuration control is obtained; and the first target quality control rule is configured as an internal quality control rule for the target quality control material. That is, when a user clicks on a rule configuration control corresponding to a quality control rule, the corresponding quality control rule is determined to be the first target quality control rule, and the first target quality control rule is configured as an internal quality control rule for the target quality control material.

Both methods enable the configuration of recommended quality control rules as the internal quality control rules for target control materials directly from the recommendation interface.

In another embodiment, configuring the first target quality control rule as the internal quality control rule of the target quality control material includes: displaying a rule configuration interface; and configuring the first target quality control rule as the internal quality control rule of the target quality control material in the rule configuration interface.

When the user triggers the rule configuration control in the recommendation interface, the display is switched to the rule configuration interface shown in FIG. 4, and in the rule configuration interface, the configuration of the quality control rule of the target quality control material is automatically completed according to the first target quality control rule configuration. Thus, the user does not need to perform manual configuration. For example, in the recommendation interface shown in FIG. 3, the user selects the recommended rule 1_3S as the recommended rule for the target quality control material. When the user clicks the rule configuration control 506, the display is switched to the rule configuration interface shown in FIG. 4, and the quality control rule 1_3S is set as the out-of-control rule for the target quality control material in the rule configuration interface.

In the embodiment, by responding to the user's trigger operation on the rule configuration control, the first target quality control rule selected on the recommendation interface will be configured on the rule configuration interface without complex manual operations. When the internal quality control rule is abnormal, the target quality control rule can be quickly determined and quickly set according to the recommended function of the quality control rule.

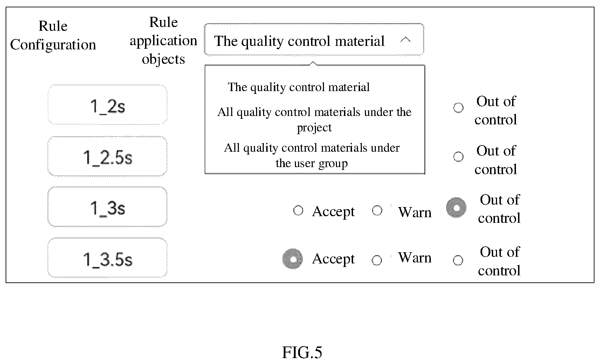

Furthermore, in addition to configuring the quality control rules for the target quality control material, the user can also expand the application objects of the target quality control rules. As shown in FIG. 5, the application object configuration area of an embodiment includes an option box in the form of a drop-down box. When the user triggers the option box, an application object list is displayed, and the application object list includes configurable application objects. As shown in FIG. 5, the application object list can include a single quality control material, or all quality control materials under a project, or all quality control materials under a user group.

Specifically, in the rule configuration interface, the first target quality control rule is configured as the internal quality control rule of the target quality control material, including: when the configuration object selected from the application object list is a target quality control material, the first target quality control rule being configured as the internal quality control rule of the target quality control material. When the configuration object selected from the application object list is the quality control material under the project to which the target quality control material belongs, the first target quality control rule is configured as the internal quality control rule of each quality control material of the project. When the configuration object selected from the application object list is the quality control material of the laboratory to which the target quality control material belongs, the first target quality control rule is configured as the internal quality control rule of each quality control material of the laboratory.

In the embodiment, a quick configuration function can be provided through the application object list, and the first target quality control rule can be applied as the target quality control material, or all quality control materials under the application project, or all quality control materials under the user group, so as to realize the quick configuration of the rule.

In one embodiment, the internal quality control rules include out-of-control rules. The rule configuration interface includes a default configuration area. The default configuration area includes a first quality control rule list. The first quality control rule list includes all quality control rules.

In the rule configuration interface, the first target quality control rule is configured as the internal quality control rule of the target quality control material, including: in the default configuration area, the first target quality control rule in the first quality control rule list is configured as the out-of-control rule of the target quality control material.

In one embodiment, the internal quality control rules include acceptance rules, warning rules and out-of-control rules. The acceptance rule indicates that when the quality control result meets certain conditions, the quality control result is considered acceptable, that is, the quality control result is under control. The warning rule indicates that when the quality control result exceeds a certain range, it is determined as a warning. The out-of-control rule indicates that when the quality control result exceeds the certain range, it is determined as out-of-control.

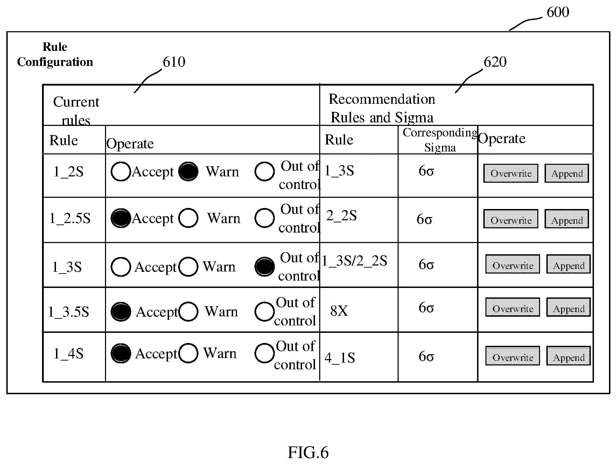

The quality control rule recommendation can be used to recommend out-of-control rules for target quality control materials. Specifically, when the user triggers the rule configuration control in the recommendation interface, the display is switched to the rule configuration interface shown in FIG. 6. As shown in FIG. 6, the rule configuration interface 600 includes a default configuration area 610, and the default configuration area 610 includes a first quality control rule list, and the first quality control rule list includes all quality control rules, and rule type operation options. The rule type operation options are used to configure the quality control rules to the corresponding rule types. For example, the rule type operation options include an acceptance option, a warning option, and an out-of-control option. When the user selects the acceptance option of a quality control rule, the quality control rule is configured as the acceptance rule for the target quality control material. When the user selects the out-of-control option of a quality control rule, the quality control rule is configured as the out-of-control rule for the target quality control material. That is, the setting of the rule type operation options can facilitate users to manually set the quality control rules for the target quality control material.

Furthermore, when the user triggers the rule configuration control in the recommendation interface, the display switches to the rule configuration interface shown in FIG. 6, and in the default configuration area, the first target quality control rule in the first quality control rule list is configured as the out-of-control rule for the target quality control material. That is, without manual operation, the first target quality control rule selected by the user in the recommendation interface is automatically configured as the out-of-control rule for the target quality control material in the default configuration area of the rule configuration interface.

Understandably, in the default configuration area of the rule configuration interface, after configuring the first target quality control material as the out-of-control rule for the target quality control material, if there is a need to modify the configuration rule, you can manually select the out-of-control option of a quality control rule to modify the out-of-control rule for the target quality control material.

In another embodiment, the rule configuration interface also includes a quick configuration area. The quick configuration area includes a second quality control rule list. The second quality control rule list includes at least one quality control recommended rule, and at least one operation control corresponding to the quick configuration method for each quality control recommended rule.

Specifically, when the user triggers the rule configuration control in the recommendation interface, the display switches to the rule configuration interface shown in FIG. 6. As shown in FIG. 6, the rule configuration interface 600 includes a default configuration area 610 and a quick configuration area 620. The default configuration area 610 includes a first quality control rule list. The first quality control rule list includes all quality control rules, and rule type operation options, and the rule type operation option is used to configure the quality control rule to the corresponding rule type. The quick configuration area 620 includes a second quality control rule list. The second quality control rule list includes at least one quality control recommendation rule, and at least one operation control corresponding to the quick configuration method for each quality control recommendation rule. In one embodiment, the second quality control rule list in the quick configuration area includes all quality control recommendation rules.

The second quality control list also includes operation controls corresponding to at least one quick configuration method for each quality control rule. In one embodiment, two quick configuration methods are provided, namely, an overwriting method and an appending method. The overwriting method means to overwrite the existing out-of-control rule with the quality control rule. The appending method means to add a new out-of-control rule. Correspondingly, operation controls corresponding to overwrite and operation controls corresponding to append are provided respectively.

The configuration method based on recommended quality control rules also includes: in response to the operation of the operation control of any quality control recommended rule in the quick configuration area, obtaining a second target quality control rule and a quick configuration method; the quick configuration method including at least one of overwriting method and appending method; if the quick configuration method is overwriting method, then in the default configuration area, the current out-of-control rule in the first quality control rule list is cancelled, and the second target quality control rule is updated to the out-of-control rule for the target quality control material; if the quick configuration method is appending, then in the default configuration area, the second target quality control rule in the second quality control rule list is added as the out-of-control rule for the target quality control material.

That is, the overwriting method overwrites the currently configured out-of-control rule of the target quality control material, and configures the second target quality control rule as a new out-of-control rule. For the target quality control material, the out-of-control rule only includes the second target quality control rule. The appending method, for the target quality control material, in addition to the currently configured out-of-control rule, also includes the appended second target quality control rule, that is, the out-of-control rule of the target quality control material includes the configured out-of-control rule and the second target quality control rule.

Compared with the conventional rule method, the quick configuration area provides a quick configuration method. By displaying a smaller number of quality control rule recommendations in the quick configuration area, it is convenient for users to filter and set the quality control rule recommendations, and synchronize to the general settings area for rule configuration, improving setting efficiency.

The above-mentioned method for recommending quality control rule is applied to the application client, by providing a recommendation interface, which displays at least one quality control recommendation rule recommended for the target quality control material, thereby eliminating the need for users to manually calculate the internal quality control rules, thereby improving the accuracy and efficiency of internal quality control rule configuration. In order to improve the confidence of the quality control recommendation rules, the recommendation interface also displays the quantitative value of the recommendation effect of each quality control recommendation rule, as well as the quality control analysis results, so that users can intuitively understand the quality control analysis process and results. By setting interactive controls such as recommendation controls, configuration areas, and rule configuration controls on the recommendation interface, the quality control rule recommendation and configuration process can be completed through interactive design.

It should be understood that, although the various steps in the flowcharts involved in the above-mentioned embodiments are displayed in sequence according to the indication of the arrows, these steps are not necessarily executed in sequence according to the order indicated by the arrows. Unless there is a clear explanation in the application, the execution of these steps is not strictly limited in order, and these steps can be executed in other orders. Moreover, at least a part of the steps in the flowcharts involved in the above-mentioned embodiments can include multiple steps or multiple stages, and these steps or stages are not necessarily executed at the same time, but can be executed at different times, and the execution order of these steps or stages is not necessarily carried out in sequence, but can be executed in turn or alternately with other steps or at least a part of the steps or stages in other steps.

Based on the same inventive concept, the embodiment of the present application also provides a quality control rule recommendation device (device for recommending quality control rule) for implementing the method for recommending quality control rule involved above. The implementation scheme for solving the problem provided by the device is similar to the implementation scheme recorded in the above method, so the specific limitations in the embodiments of one or more quality control rule recommendation devices provided below can refer to the limitations of the method for recommending quality control rule above, and will not be repeated here.

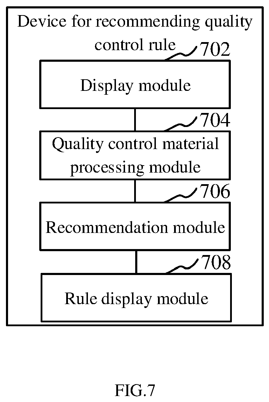

In one embodiment, as shown in FIG. 7, a device for recommending quality control rule is provided, which is applied to an application client and includes following modules.

A display module 702, is used to display a recommendation interface, which includes an information display area and a rule recommendation area.

A quality control material processing module 704, is used to display the target quality control material in the information display area.

A recommendation module 706, is used to obtain at least one quality control recommendation rule recommended for the target quality control material.

A rule display module 708, is used to display at least one quality control recommendation rule of the target quality control material in the rule recommendation area of the recommendation interface.

In another embodiment, a basis acquisition module is further included in the device to acquire the recommendation rationale of each quality control recommendation rule.

The rule display module is also used to display at least one quality control recommendation rule for the target quality control material and the recommendation rationale corresponding to each quality control recommendation rule in the rule recommendation area.

In another embodiment, the information display area includes a plurality of levels of attribute selection bars for the quality control material.

The rule display module is used to respond to the selection operation of the attribute selection bar of each level, and display the selected attributes of each level in the attribute selection bar of each level; and determine the target quality control material according to the selected attributes of each level.

In another embodiment, the target quality control material includes at least one of the following type:
a first type: the target quality control material is a quality control material with abnormal internal quality control rules;
a second type: the target quality control material is a quality control material whose sigma value of internal quality control is lower than the first threshold;
a third type: the target quality control material is a quality control material whose sigma value of internal quality control is lower than the second threshold and has not increased within a preset time period.

In another embodiment, the recommendation interface further includes a recommendation control.

The recommendation module is used to obtain at least one quality control recommendation rule for the target quality control material in response to a trigger operation on a recommendation control in a recommendation interface.

In another embodiment, the recommendation interface further includes a parameter configuration area. The parameter configuration area includes a plurality of quality control parameters to be set.

The device also includes a setting module. The setting module is used to obtain the configuration operation in the parameter configuration area of the recommendation interface and obtain the setting multiple quality control parameters.

The recommendation module is further used to obtain at least one quality control recommendation rule for the target quality control material based on the plurality of quality control parameters in response to a trigger operation on the recommendation control in the recommendation interface.

In another embodiment, the recommendation interface further includes an analysis result area.

The device also includes:
an analysis result display module, is used to obtain the quality control analysis results of the quality control recommendation analysis, the quality control analysis results including efficacy function graphs of plurality of quality control rules and operation process specification graphs, the quality control analysis results being used to determine at least one quality control recommendation rule; display the quality control analysis results in the analysis result area.

In another embodiment, the recommendation interface also includes a rule configuration control.

The device also includes a configuration module. The configuration module is used to obtain a first target quality control rule selected from at least one quality control recommendation rule; in response to a trigger operation on a rule configuration control in a recommendation interface, configure the first target quality control rule as an internal quality control rule for a target quality control material.

In another embodiment, the recommendation interface further includes a plurality of rule configuration controls corresponding to each quality control recommendation rule.

The device also includes a configuration module. The configuration module is used to obtain a first target quality control rule corresponding to the rule configuration control in response to a triggering operation on the rule configuration control of the recommendation interface; and configure the first target quality control rule as an internal quality control rule for the target quality control material.

The configuration module is used to display a rule configuration interface; configure the first target quality control rule as the internal quality control rule of the target quality control material in the rule configuration interface.

In another embodiment, the internal quality control rules include out-of-control rules. The rule configuration interface includes a default configuration area. The default configuration area includes a first quality control rule list. The first quality control rule list includes all quality control rules.

The configuration module is used to configure the first target quality control rule in the first quality control rule list as the out-of-control rule for the target quality control material in the default configuration area.

In another embodiment, the configuration interface also includes a quick configuration area. The quick configuration area includes a second quality control rule list. The second quality control rule list includes at least one quality control recommendation rule, and at least one operation control corresponding to the quick configuration method for each quality control recommendation rule.

The configuration module is further used to obtain a second target quality control rule and a quick configuration method in response to an operation of an operation control of any quality control recommendation rule in the quick configuration area. The quick configuration method includes at least one of overwriting method and appending method.

If the quick configuration method is the overwriting method, then the configuration module cancels current out-of-control rule in the first quality control rule list, and updates the second target quality control rule to the out-of-control rule of the target quality control material in the default configuration area.

If the quick configuration method is the appending method, then the configuration module adds the second target quality control rule in the second quality control rule list as the out-of-control rule for the target quality control material in the default configuration area.

Each module in the above-mentioned quality control rule recommendation device can be implemented in whole or in part by software, hardware and their combination. Each of the above-mentioned modules can be embedded in or independent of the processor in the computer device in the form of hardware, or can be stored in the memory of the computer device in the form of software, so that the processor can call and execute the corresponding operations of each of the above modules.

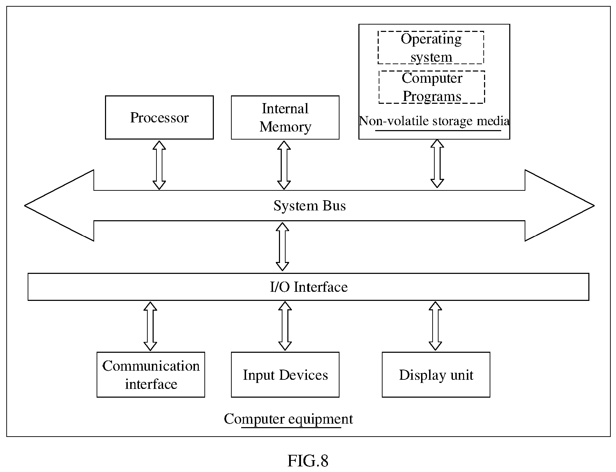

In one embodiment, a computer device is provided, which may be a terminal, and its internal structure diagram may be shown in FIG. 8. The computer device includes a processor, a memory, a communication interface, a display screen, and an input device connected through a system bus. In one embodiment, the processor of the computer device is used to provide computing and control capabilities. The memory of the computer device includes a non-volatile storage medium and an internal memory. The non-volatile storage medium stores an operating system and a computer program. The internal memory provides an environment for the operation of the operating system and the computer program in the non-volatile storage medium. The communication interface of the computer device is used to communicate with an external terminal in a wired or wireless manner, and the wireless manner can be implemented through WIFI, a mobile cellular network, near field communication (NFC) or other technologies. When the computer program is executed by the processor, a method for recommending a quality control rule is implemented. The display screen of the computer device may be a liquid crystal display screen or an electronic ink display screen, and the input device of the computer device may be a touch layer covered on the display screen, or a key, trackball or touchpad provided on the housing of the computer device, or an external keyboard, touchpad or mouse, etc.

Those skilled in the art will understand that the structure shown in FIG. 8 is merely a block diagram of a partial structure related to the solution of the present application, and does not constitute a limitation on the computer device to which the solution of the present application is applied. The specific computer device may include more or fewer components than shown in the figures, or combine certain components, or have a different arrangement of components.

In one embodiment, a computer device is provided, including a memory and a processor, wherein a computer program is stored in the memory, and when the processor executes the computer program, the steps of the method for recommending quality control rules of the above-mentioned embodiments are implemented.

In one embodiment, a computer-readable storage medium is provided, on which a computer program is stored. When the computer program is executed by a processor, the steps of the method for recommending quality control rules in the above-mentioned embodiments are implemented.

In one embodiment, a computer program product is provided, including a computer program, which, when executed by a processor, implements the steps of the method for recommending quality control rules in the above-mentioned embodiments.

A person of ordinary skill in the art can understand that all or part of the processes in the above-mentioned embodiment method can be completed by instructing the relevant hardware through a computer program, and the computer program can be stored in a non-volatile computer-readable storage medium. When the computer program is executed, it can include the processes of the embodiments of the above-mentioned methods. In one embodiment, any reference to the memory, database or other medium used in the embodiments provided in the present application can include at least one of non-volatile and volatile memory. Non -volatile memory can include read-only memory (ROM), magnetic tape, floppy disk, flash memory, optical memory, high-density embedded non-volatile memory, resistive random access memory (ReRAM), magnetic random access memory (MRAM), ferroelectric random access memory (FRAM), phase change memory (PCM), graphene memory, etc. Volatile memory can include random access memory (RAM) or external cache memory, etc. As an illustration and not limitation, RAM can be in various forms, such as static random access memory (SRAM) or dynamic random access memory (DRAM). The database involved in each embodiment provided in this application may include at least one of a relational database and a non-relational database. Non-relational databases may include distributed databases based on blockchains, etc., but are not limited to this. The processor involved in each embodiment provided in this application may be a general-purpose processor, a central processing unit, a graphics processor, a digital signal processor, a programmable logic unit, a data processing logic unit based on quantum computing, etc., but are not limited to this.

The technical features of the above embodiments may be combined arbitrarily. To make the description concise, not all possible combinations of the technical features in the above embodiments are described. However, as long as there is no contradiction in the combination of these technical features, they should be considered to be within the scope of this specification.

The above-described embodiments only express several implementation methods of the present application, and the descriptions thereof are relatively specific and detailed, but they cannot be understood as limiting the scope of the present application. It should be pointed out that, for a person of ordinary skill in the art, several variations and improvements can be made without departing from the concept of the present application, and these all belong to the protection scope of the present application. Therefore, the protection scope of the present application shall be subject to the attached claims.

## Claims

1. A method for recommending quality control rules, **characterized in that** the method comprises:
displaying a recommendation interface, and the recommendation interface comprising an information display area and a rule recommendation area;
displaying a target quality control material in the information display area;
obtaining at least one quality control recommendation rule recommended for the target quality control material;
displaying the at least one quality control recommendation rule for the target quality control material in the rule recommendation area of the recommendation interface.

2. The method according to claim 1, **characterized in that** the method further comprises:
obtaining a recommendation rationale for each quality control recommendation rule;
displaying the at least one quality control recommendation rule for the target quality control material in the rule recommendation area of the recommendation interface, comprises:
displaying the at least one quality control recommendation rule for the target quality control material and the recommendation rationale corresponding to each quality control recommendation rule in the rule recommendation area.

3. The method according to claim 1, **characterized in that** the information display area comprises a plurality of levels of attribute selection bars of a quality control material;
displaying the target quality control material in the information display area, comprises:
in response to a selection operation on the attribute selection bar of each level, displaying selected attributes of each level in the attribute selection bar of each level;
determine the target quality control material according to the selected attributes of each level.

4. The method according to claim 1, **characterized in that** the target quality control material comprises at least one of the following types:
a first type, indicating that the target quality control material is a quality control material with abnormal internal quality control rules;
a second type, indicating that the target quality control material is the quality control material whose sigma value of internal quality control is lower than a first threshold;
a third type, indicates that the target quality control material is the quality control material whose sigma value of internal quality control is lower than a second threshold and has not increased within a preset time period.

5. The method according to claim 1, **characterized in that** the recommendation interface also comprises a recommendation control, obtaining at least one quality control recommendation rule recommended for the target quality control material, comprises:
in response to a trigger operation on the recommendation control in the recommendation interface, obtaining the at least one quality control recommendation rule for the target quality control material.

6. The method according to claim 5, **characterized in that** the recommendation interface further comprises a parameter configuration area, and the parameter configuration area comprises a plurality of quality control parameters to be set;
the method further comprises:
obtaining a configuration operation in the parameter configuration area of the recommendation interface, and obtaining a plurality of the quality control parameters;
in response to the trigger operation on the recommendation control in the recommendation interface, obtaining the at least one quality control recommendation rule for the target quality control material, comprises:
in response to a triggering operation on the recommendation control in the recommendation interface, obtaining the at least one quality control recommendation rule for the target quality control material based on the plurality of quality control parameters.

7. The method according to claim 1 or 2, **characterized in that** the recommendation interface further comprises an analysis result area;
the method further comprises:
obtaining a quality control analysis result for quality control recommendation analysis; and the quality control analysis result comprising a function graph of a plurality of quality control rules and an operation process specification graph; and the quality control analysis result being used to determine the at least one quality control recommendation rule;
displaying the quality control analysis result in the analysis result area.

8. The method according to claim 1, **characterized in that** the recommendation interface further comprises a rule configuration control;
the method further comprises:
obtaining a first target quality control rule selected from the at least one quality control recommendation rule;
in response to a triggering operation on the rule configuration control in the recommendation interface, configuring the first target quality control rule as an internal quality control rule for the target quality control material.

9. The method according to claim 1, **characterized in that** the recommendation interface further comprises a plurality of rule configuration controls corresponding to each of quality control recommendation rules;
the method further comprises:
in response to a triggering operation on a rule configuration control of the recommendation interface, obtaining a first target quality control rule corresponding to the rule configuration control;
configuring the first target quality control rule as an internal quality control rule of the target quality control material.

10. The method according to claim 8 or 9, **characterized in that** configuring the first target quality control rule as the internal quality control rule of the target quality control material comprises:
displaying a rule configuration interface;
configuring the first target quality control rule as the internal quality control rule of the target quality control material in the rule configuration interface.

11. The method according to claim 10, **characterized in that** the internal quality control rule comprises an out-of-control rule, and the rule configuration interface comprises a default configuration area, and the default configuration area comprises a first quality control rule list, and the first quality control rule list comprises all quality control rules;
configuring the first target quality control rule as the internal quality control rule of the target quality control material in the rule configuration interface, comprises:
configuring the first target quality control rule in the first quality control rule list as the out-of-control rule for the target quality control material in the default configuration area.

12. The method according to claim 11, **characterized in that** the rule configuration interface further comprises a quick configuration area, and the quick configuration area comprises a second quality control rule list, and the second quality control rule list comprises at least one quality control recommendation rule, and at least one operation control corresponding to the quick configuration method for each of the quality control recommendation rules, the method further comprises:
in response to an operation on the operation control of any of the quality control recommendation rules in the quick configuration area, obtaining a second target quality control rule and a quick configuration method, and the quick configuration method comprising at least one of an overwriting method and an appending method;
in response that the quick configuration method is the overwriting method, then in the conventional configuration area, cancelling current out-of-control rule in the first quality control rule list, and updating the second target quality control rule to the out-of-control rule of the target quality control material;
in response that the quick configuration method is the appending method, then in the conventional configuration area, appending the second target quality control rule in the second quality control rule list as the out-of-control rule for the target quality control material.

13. A device for recommending quality control rule, **characterized in that** the device comprises:
a display module, used to display a recommendation interface, and the recommendation interface comprising an information display area and a rule recommendation area;
a quality control material processing module, used to display a target quality control material in the information display area;
a recommendation module, used to obtain at least one quality control recommendation rule recommended for the target quality control material;
a rule display module, used to display at least one quality control recommendation rule of the target quality control material in the rule recommendation area of the recommendation interface.

14. A computer equipment, comprising a memory and a processor, **characterized in that** the memory stores computer programs, wherein the processor implements steps of a method according to any one of claims 1 to 12 when executing the computer programs.
